# EUROPÄISCHE PATENTANMELDUNG

(11) **EP 1 389 710 A2**
(43) Veröffentlichungstag der Anmeldung: **18.02.2004**
(21) Anmeldenummer: 03017671.3
(22) Anmeldetag: 14.08.2003
(51) Int. Cl.: F21S 8/00, F21V 8/00, A61B 19/00

(54) **Anordnung zum Beleuchten eines dentalen Operationsfeldes**

(30) Priorität: 15.08.2002 DE 10237575
(71) Anmelder: OLPE Jena GmbH, 07745 Jena (DE)
(72) Erfinder: Jubold, Lutz, 07745 Jena (DE); Hornung, Randy, 07747 Jena (DE)
(74) Vertreter: Freitag, Joachim, Dipl.-Phys.

(57) **Zusammenfassung**

Die Erfindung betrifft eine Anordnung zum Beleuchten eines dentalen Operationsfeldes.

Die Aufgabe, eine neue Möglichkeit zum Beleuchten eines für dentale Anwendungen definiert ausgeleuchteten Beleuchtungsfeldes zu finden, die eine intensive Beleuchtung des vorgeschriebenen Beleuchtungsfeldes mit geringer Wärmestrahlung und mehr Sicherheit vor Leuchtmittelexplosion gestattet, wird erfindungsgemäß gelöst, indem bei einer Anordnung zum Beleuchten eines Dentalarbeitsplatzes, die Lichtquelle einen außerhalb des Hauptreflektors (1) separat angeordneten Lichtgenerator (4) aufweist, wobei das Licht des Lichtgenerators (4) über einen Lichtleiter (41) übertragen wird und der Lichtleiter (41) von hinten in den Innenraum des Hauptreflektors (1) geführt ist, und eine übliche Abschatteinrichtung für direktes Licht der Lichtquelle als ein Sekundärspiegel (3) ausgeführt ist, wobei der Sekundärspiegel (3) erhaben geformt und auf der optischen Achse (11) des Hauptreflektors (1) so angeordnet ist, dass das aus dem Lichtleiter (41) austretende Lichtbündel vollständig vom Sekundärspiegel (3) auf den Hauptreflektor (1) reflektiert wird.

## Beschreibung

Die Erfindung betrifft eine Anordnung zum Beleuchten eines Operationsfeldes, insbesondere für den Dentalbereich, bei der ein kleines, definiert ausgeleuchtetes Leuchtfeld in geringer Entfernung von der Lichtquelle benötigt wird. Sie ist vorzugsweise in der Dentalmedizin und Kieferchirurgie zur Beleuchtung der Mundhöhle geeignet, bietet jedoch auch andere Anwendungsmöglichkeiten, bei denen es auf hohe Beleuchtungsstärken in einer begrenzten Fläche und die Vermeidung von Schlagschattenwurf ankommt.

In der Dentalmedizin oder Kieferchirurgie werden hohe Anforderungen an eine lichistarke Beleuchtung einer definierten Ebene gestellt. Die Eigenschaften des benötigten Lichtfeldes sind in Vorschriften nach EN ISO 9680 (1996) verankert. Damit soll sichergestellt werden, dass eine Lichtquelle auf ein 700 mm entferntes Beleuchtungsfeld (Mundhöhle, Behandlungszone E3 nach EN ISO 9680) abgebildet wird, wobei ein rechteckiges Beleuchtungsfeld mit definierten Abmaßen erzeugt wird. Weiterhin soll das Beleuchtungsfeld eine sehr hohe und gleichmäßig verteilte Beleuchtungsstärke mit sehr geringem Schlagschattenwurf und genauen Farbwiedergabeeigenschaften (optimale Farbtemperatur bei 5500, zulässig nach DIN-Vorschrift sind 4500 bis 6000 K) aufweisen und durch eine scharfe Begrenzung des Beleuchtungsfeldes eine Blendung des Patienten vermieden werden. Wünschenswert ist des Weiteren eine geringe Strahlungswärme im Beleuchtungsfeld sowie im Aufenthaltsbereich des Zahnarztes.

Der bekannt gewordene Stand der Technik der Dentalleuchten ist recht vielgestaltig. So ist bereits aus der deutschen Auslegeschrift DE 14 66 879 eine ärztliche Mundhöhlen-Beleuchtungseinrichtung bekannt geworden, die eine rechteckig begrenzte Beleuchtungsfläche erzeugt. Hier wird das Licht einer Lichtquelle durch einen Reflektor in einem äußeren Brennpunkt gesammelt. Der Reflektor besitzt in Ebenen senkrecht zur Längsachse der Lichtquelle einen ellipsenförmigen Querschnitt und in den Ebenen, die parallel zur Längsachse liegen, einen parabelförmigen Querschnitt. Zur Justierung des Beleuchtungsfeldes ist die Lichtquelle in ihrer Halterung zum inneren Brennpunkt verschiebbar.
Aus der DE 3843253 C2 ist eine zahnärztliche Beleuchtungseinheit für ein in der Horizontalen stärker als in der Vertikalen ausgedehntes Beleuchtungsfeld, die einen ellipsoidförmigen Reflektor aufweist, in dessen inneren Brennpunkt die Lichtquelle und in deren äußeren Brennpunkt eine Blende angeordnet ist, so dass mittels abbildender Elemente sowohl die Lichtquelle als auch die Blende in die Beleuchtungsebene abgebildet werden. Nachteilig sind hierbei vor allem der starke Schlagschattenwurf und die hohe Wärmestrahlung im Lichtfeld.

Zur Reduzierung der Wärmeentwicklung einer Dentalleuchte ist in der DE 100 34 594 A1 eine zahnärztliche Behandlungsleuchte offenbart, die zur Erzeugung eines Beleuchtungsfeldes mit vorgegebener Größe, Beleuchtungsstärke und Homogenität und Farbtemperatur eine Lichtquelle aus mehreren benachbart angeordneten LED angibt. Dabei sind zur Bündelung oder Aufweitung des Strahlengangs optische Bauelemente vorhanden, die den LED einzeln oder gruppenweise zugeordnet sind. In einer Variante ist auch eine indirekte Beleuchtung über einen herkömmlichen Reflektor beschrieben. Nachteilig an dieser Lösung ist in jedem Fall die Kostenseite, wenn man bei der Vielzahl der LED eine homogene Beleuchtung und (wegen des begrenzten Emissionsspektrums herkömmlicher LED) gute Farbwiedergabeeigenschaften erreichen will. Problematisch ist zudem bei indirekter Beleuchtung die Reinigung und Desinfektion sowie ein Leuchtmittelaustausch.
Des Weiteren sind für Dentalarbeitsplätze Beleuchtungen unter Verwendung von optischen Fasern bekannt geworden, von denen die Patentschrift US 3,758,951 und die Offenlegungsschrift DE 38 32 811 A1 beispielhaft angeführt sein sollen. Diese Lösungsansätze enthalten ein fiberoptisches Beleuchtungssystem, bei dem keine großflächige Beleuchtungsebene erzeugt wird, sondern das von einer entfernt befindlichen Lichtquelle erzeugte Licht mittels einer optischen Faser auf das Behandlungsziel eines zahnärztlichen Handteils (Bohrer, Spiegel etc.) gerichtet wird, indem die optische Faser in geeigneter Weise an dem Handteil befestigt ist. Für die großflächige Beleuchtung der Mundhöhle fehlt es diesen Lösungen wegen der begrenzten Zweckbestimmung vor allem an Anregungen für die technische Realisierung einer geeigneten Strahlaufweitung.

Der Erfindung liegt die Aufgabe zugrunde, eine neue Möglichkeit zum Beleuchten eines Operationsfeldes, insbesondere eines für dentale Anwendungen definiert ausgeleuchteten Beleuchtungsfeldes in festgelegter Entfernung zu finden, die eine intensive Beleuchtung des vorgeschriebenen Beleuchtungsfeldes mit geringer Wärmestrahlung und mehr Sicherheit vor Leuchtmittelexplosion gestattet.

Erfindungsgemäß wird die Aufgabe bei einer Anordnung zum Beleuchten eines dentalen Operationsfeldes, bei der eine Lichtquelle in einem konkav geformten Hauptreflektor zum Formen eines definierten Beleuchtungsfeldes angeordnet ist, eine Abschatteinrichtung zur Vermeidung direkter Lichtabstrahlung von der Lichtquelle ins Beleuchtungsfeld sowie Halterungs- und Bewegungselemente, die am Hauptreflektor zur Ausrichtung des Beleuchtungsfeldes angebracht sind, vorhanden sind, dadurch gelöst, dass die Lichtquelle eine Austrittsfläche eines Lichtleiters ist, der von hinten in den Innenraum des Hauptreflektors geführt ist, wobei der Lichtleiter mit einem außerhalb des Hauptreflektors separat angeordneten Lichtgenerator in Verbindung steht und die Austrittsfläche des Lichtleiters mit ihrer Flächennormalen auf die Abschatteinrichtung ausgerichtet ist, und dass die Abschatteinrichtung als Sekundärspiegel ausgeführt ist, wobei der Sekundärspiegel erhaben geformt und auf der optischen Achse des Hauptreflektors angeordnet ist, so dass ein aus dem Lichtleiter austretendes Lichtbündel vollständig vom Sekundärspiegel reflektiert und vom Hauptreflektor zu dem definierten Beleuchtungsfeld geformt wird.

Vorzugsweise hat der Sekundärspiegel die Form eines konvexen sphärischen Spiegels, wobei der sphärische Spiegel zwischen dem Scheitelpunkt und dem Brennpunkt nahe dem Brennpunkt des Hauptreflektors angeordnet ist, so dass das aus dem Lichtleiter austretende Licht den Hauptreflektor im Wesentlichen vollständig ausleuchtet.
In einer anspruchsvolleren Variante ist der Sekundärspiegel ein sphärisch-parabolischer Spiegel, wobei dieser sphärisch-parabolische Spiegel in einer Schnittebene, die der Längsausdehnung des Hauptreflektors entspricht, parabolisch und in der dazu orthogonalen Schnittebene, die der kürzeren Querausdehnung des Hauptreflektors entspricht, sphärisch geformt ist, und zwischen dem Scheitelpunkt und dem Brennpunkt des Hauptreflektors so angeordnet ist, dass das aus dem Lichtleiter austretende Licht den Hauptreflektor im Wesentlichen vollständig ausleuchtet.
In einer weiteren vorteilhaften Gestaltungsform ist der Sekundärspiegel ein konvexer zylindrischer Spiegel, wobei der zylindrische Spiegel einen gegenüber seiner Höhe im Wesentlichen gleich großen Durchmesser aufweist und mit seiner Achse parallel zur kurzen Querrichtung des Hauptreflektors so angeordnet ist, dass eine als Scheitel wirkende Mantellinie des zylindrischen Spiegels zwischen Scheitelpunkt und Brennpunkt des Hauptreflektors nahe dessen Brennpunkt angeordnet ist.
Eine fertigungstechnisch einfachere Ausführung ergibt sich für einen kegelförmigen Spiegel als Sekundärspiegel, wobei der kegelförmige Spiegel mit seiner Spitze zwischen dem Scheitelpunkt und dem Brennpunkt des Hauptreflektors so angeordnet ist, dass das aus dem Lichtleiter austretende Lichtbündel aufgrund seiner kegelförmig divergierenden Bündelform auf den Hauptreflektor verteilt wird.
Der Lichtleiter wird vorteilhaft koaxial zur gemeinsamen optischen Achse des Hauptreflektors durch den Hauptreflektor hindurch geführt, wobei der Sekundärspiegel ebenfalls koaxial zum Hauptreflektor angeordnet ist.
Der Lichtleiter kann aber auch zweckmäßig außerhalb der optischen Achse des Hauptreflektors, z.B. entlang einer Schnittebene, die den Hauptreflektor symmetrisch teilt, seinen Durchstoßpunkt durch den Hauptreflektor haben und auf den Sekundärspiegel gerichtet sein. Ebenfalls geeignet ist eine Variante, bei der der Lichtleiter außerhalb der optischen Achse und außerhalb der Spiegelfläche des Hauptreflektors, z.B. in Richtung der kurzen Querausdehnung des Hauptreflektors seitlich neben dem Hauptreflektor auf den Sekundärspiegel gerichtet ist. In dieser speziellen Variante ist der Sekundärspiegel, insbesondere wenn er die Form einer flachen Kegelmantelfläche aufweist, in seiner Mittelachse gegenüber der optischen Achse des Hauptreflektors zum Einkopplungspunkt des Lichtleiters hin geneigt.

Um für die Ausrichtung des Beleuchtungsfeldes keinen unnötigen Biegewiderstand überwinden zu müssen, ist der Lichtleiter vorteilhaft aus einzelnen Fasern zusammengesetzt. Der Lichtleiter besteht dazu vorteilhaft aus einzelnen Kunststofffasern. Dieses aus einzelnen Fasern zusammengesetzte Faserbündel ermöglicht es vorteilhaft, Fasern von wenigstens zwei Lichtgeneratoren oder einem Lichtgenerator mit mehreren Leuchtmitteln zu einem Lichtleiter zur Einkopplung am Hauptreflektor zusammenzufügen.
Bei einer solchen Zusammenführung von Faserbündeln aus mehreren simultan betriebenen Lichtgeneratoren können vorteilhaft sogar Halogenlampen als Leuchtmittel eingesetzt werden, die - einzeln betrieben - infolge der Dämpfung bei Verwendung eines langen Lichtleiters nicht die notwendige Lichtleistung erbringen würden.
Wenn der Lichtleiter eine große Entfernung von einem Lichtgenerator mit nur einem Leuchtmittel dem Hauptreflektor überbrücken muss, wird als Leuchtmittel im Lichtgenerator zweckmäßig eine Gasentladungslampe, vorzugsweise eine Quecksilberdampf-, Natriumdampf- oder Xenonlampe verwendet.
Für den Fall, dass der Lichtleiter nur eine geringe Länge aufweist, weil der Lichtgenerator in nur geringer Entfernung außerhalb des Hauptreflektors, z.B. am herkömmlichen Schwenkarm zum horizontalen Ausrichten des Hauptreflektors, angebracht ist, kann als Leuchtmittel im Lichtgenerator ein herkömmlicher Halogenstrahler mit Reflektor und nachgeordnetem IR-Filter eingesetzt werden. Bei dieser Anbringung des Lichtgenerators am Schwenkarm des Hauptreflektors ist dessen optische Achse vorteilhaft bis zu 30° abwärts zum Hauptreflektor geneigt angebracht, um die Krümmung des Lichtleiters zu minimieren.
In einer besonders lichtstarken und kostengünstigen Variante ist der Lichtgenerator unmittelbar hinter dem Hauptreflektor entlang der verlängerten optischen Achse des Hauptreflektors angebracht, wobei als Lichtleiter ein starrer Lichtleitstab eingesetzt ist und das Gehäuse des Lichtgenerators mit dem Reflektorträger des Hauptreflektors schwenkbar ist. Mit dem Lichtleitstab, der aus einer Vielzahl verschmolzener Glasfasern zusammengesetzt ist, erreicht man eine bis zu 45 % höhere Lichttransmission gegenüber herkömmlichen flexiblen Faserbündeln.

Die Erfindung basiert auf der Überlegung, dass bei den herkömmlichen leistungsfähigen Dentalleuchten ein direkt in der Beleuchtungseinheit befindliches Leuchtmittel (z.B. Halogenlampe) bis zu 80 % seiner Leistung als Wärmestrahlung abstrahlt. Selbst die Verwendung eines Kaltlichtreflektors, welcher vornehmlich Anteile des sichtbaren Lichtes reflektiert und Wärmestrahlung transmittiert, bringt nur eine Linderung, da ein Teil der Wärmestrahlung trotzdem reflektiert wird und eine direkte Erwärmung der Raumluft in der Umgebung des Leuchtmittels und somit in nur geringer Entfernung (z.B. 700 mm) von Operateur und Patient nicht verhindert werden kann. Außerdem haben die herkömmlichen Halogenstrahler als Leuchtmittel zusätzlich beim Durchbrennen des Glühfadens ein nicht zu unterschätzendes Risiko des berstenden Glaskolbens, dessen Splitter den Zahnarzt und den Patienten gefährden.
Die Erfindung löst dieses Problem, indem mittels eines Lichtleiters ein leicht divergentes kegelförmiges Lichtbündel über einen Sekundärspiegel, der zugleich die Funktion einer in herkömmlichen Dentalleuchten vorhandenen Abschattblende für die Lichtquelle übernimmt, in den üblichen Hauptreflektor (Kaltlichtreflektor) einer Dentalleuchte eingekoppelt und verteilt wird.
Wärmeentwicklung und Explosionsgefahr sind an einen von der Beleuchtungsoptik der Dentalleuchte entfernten Ort verlagert, so dass auch beliebig gestaltete Kaltlicht-Generatoren eingesetzt werden können, die im Zusammenwirken mit dem Lichtleiter und dessen Einkopplung in eine herkömmliche Dentalleuchte (Hauptreflektor mit Handhabungselementen) eine ideale Kaltlicht-Beleuchtung eines Operationsfeldes ergeben.

Die erfindungsgemäße Anordnung ermöglicht die Beleuchtung eines für dentale Anwendungen definierten Beleuchtungsfeldes in vorbestimmter Entfernung, die bei intensiver Beleuchtung eine sehr geringe Wärmestrahlung und einen sicheren Schutz vor Splittern bei Explosion des Leuchtmittels gewährleistet. Durch ein über Lichtleiter vermittelte Einkopplung des Lichts von einem außerhalb des Hauptreflektors angeordneten Lichtgenerator sind auch langlebige Bogenentladungslampen einsetzbar, die eine höhere Farbtemperatur aufweisen, und einfache Halogenstrahler, die bezüglich ihrer Berstgefahr vollständig gegenüber dem Beleuchtungsfeld der Dentalleuchte abgekapselt sind.

Die Erfindung soll nachstehend anhand von Ausführungsbeispielen näher erläutert werden. Die Zeichnungen zeigen:
- Fig.1:: eine Prinzipansicht der erfindungsgemäßen Anordnung ohne Lichtgenerator,
- Fig. 2:: zwei Schnittzeichnungen einer Ausführungsform der Erfindung mit parabolisch-sphärischem Sekundärspiegel,
- Fig. 3:: eine Variante der Erfindung mit zylindrischem Sekundärspiegel in zwei Schnittdarstellungen,
- Fig. 4:: eine Variante der Erfindung mit sphärischem Sekundärspiegel,
- Fig. 5:: eine Variante der Erfindung mit kegelförmigem Sekundärspiegel,
- Fig. 6:: eine Darstellung des Lichtgenerators, der das Licht durch eine Lichtleiterendfläche als Lichtquelle in den Hauptreflektor einbringt,
- Fig. 7:: eine Darstellung mit zwei simultan betriebenen Lichtgeneratoren, die durch Lichtleiter aus verwobenen Fasern zusammengeführt sind,
- Fig. 8:: eine weitere Variante der Erfindung mit kurzer Lichtleitfaser und kegelförmigem Sekundärspiegel,
- Fig. 9:: eine kostengünstige und lichtstarke Variante der Erfindung mit starrem Lichtleitstab und kegelförmigem Sekundärspiegel.

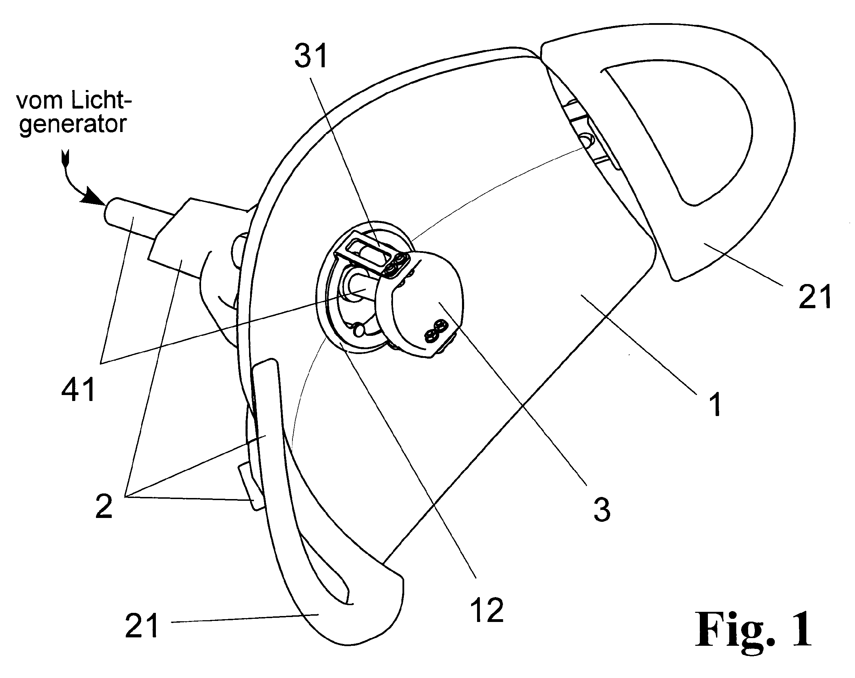

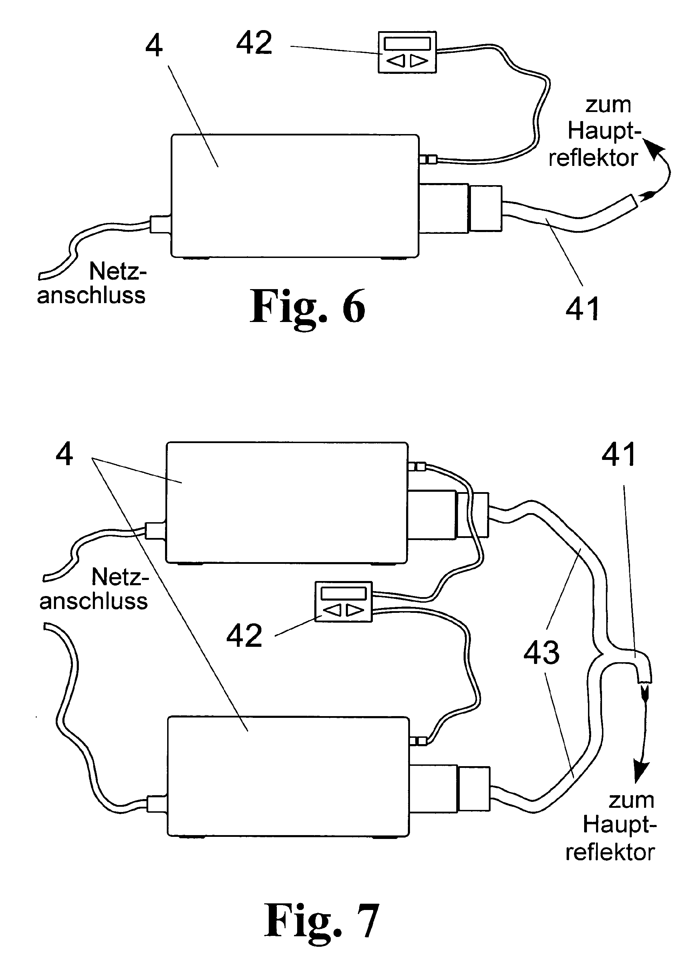

Die erfindungsgemäße Anordnung besteht - wie in Fig. 1 perspektivisch dargestellt - in ihrem Grundaufbau aus einem Hauptreflektor 1, der mit Halterungs- und Bewegungselementen 2 sowie Handgriffen 21 ausgestattet ist, einem Sekundärspiegel 3 mit Halterung 31 zur Gewährleistung einer definierten Lage gegenüber dem Hauptreflektor 1 und einem Lichtgenerator 4, von dem in Fig. 1 lediglich ein Terminal in Form eines zugeführten Lichtleiters 41 gezeigt ist und dessen übrige Komponenten nur in den Figuren 6, 7, 8 und 9 dargestellt sind.

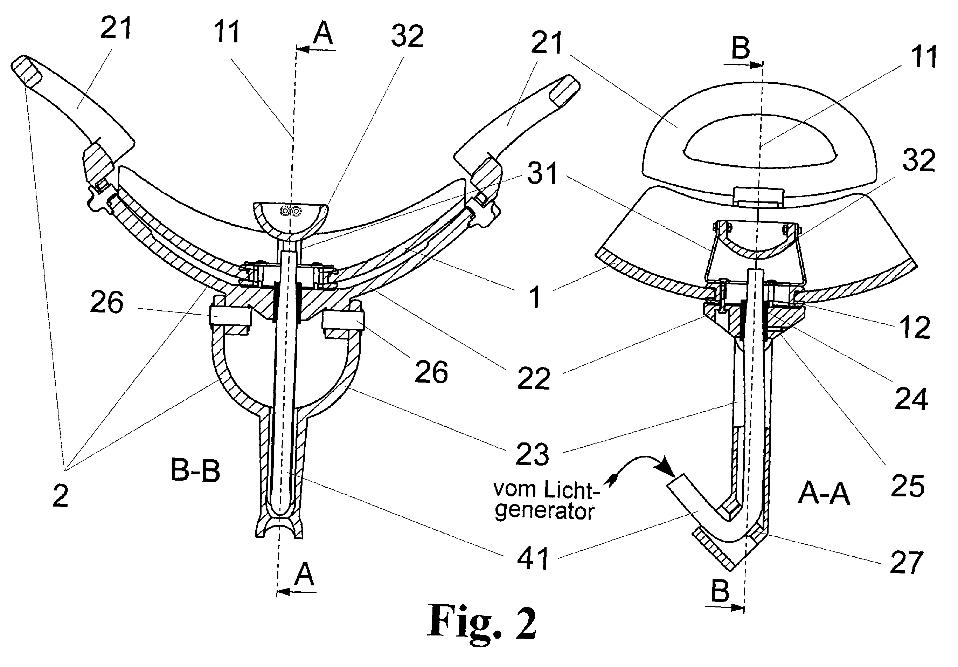

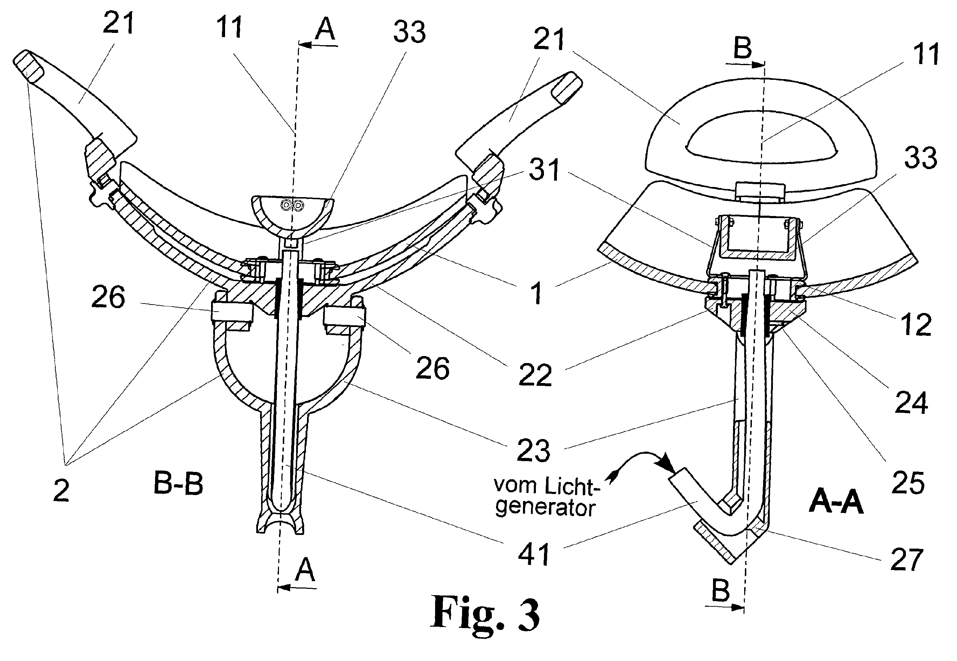

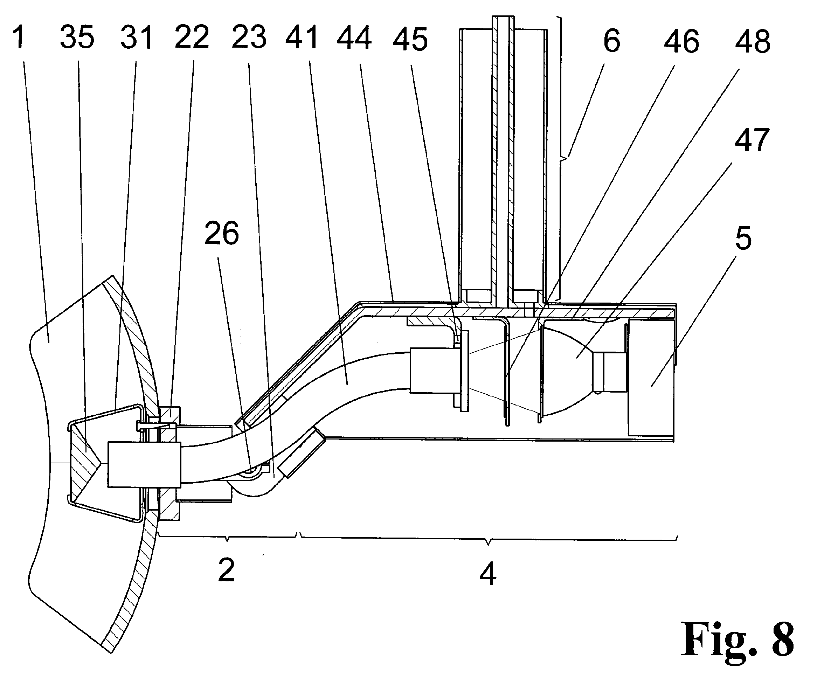

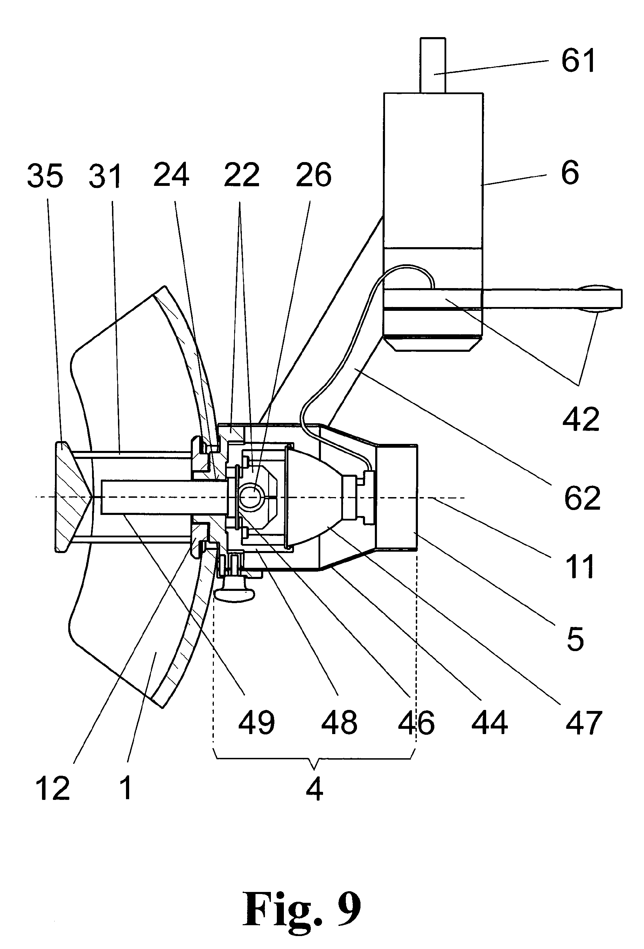

Der Hauptreflektor 1 entspricht dabei den derzeit bei den herkömmlichen Dentalleuchten eingesetzten Kaltlichtreflektoren (z.B. Dentalreflektor SUPRAX® 8488, Hersteller: Schott AUER), die vorzugsweise eine elliptisch-parabolische Hohlspiegelform aufweisen, wobei der Hauptreflektor 1 in der (horizontalen) Längsrichtung eine parabolische und der (vertikalen) kürzeren Querrichtung eine elliptische Schnittform aufweist, um in bestimmter Entfernung (700 mm von äußerster Begrenzung der Dentallampe) ein definiert geformtes, nahezu homogen ausgeleuchtetes Beleuchtungsfeld zu erhalten.
Anstelle einer bei herkömmlichen Dentalleuchten zentral angeordneten Abschattblende, die dort das im Brennpunkt des Hauptreflektors 1 angebrachte Leuchtmittel (Halogenlampe) gegen direkte Lichtaussendung in das Beleuchtungsfeld abschirmte, ist gemäß der Erfindung ein Sekundärspiegel 3 angeordnet, der ein aus dem Lichtleiter 41 divergent (kegelförmig) austretendes Lichtbündel auf den Hauptreflektor 1 ablenkt und weitgehend gleichmäßig verteilt. Der Sekundärspiegel 3 ist zur Austrittsfläche des Lichtleiters 41 erhaben geformt und wird - angepasst an seine Funktion - in gleicher Weise wie die herkömmliche Abschattblende mittels einer Halterung 31 konzentrisch zur Spiegelachse des Hauptreflektors 1 gehalten.
Der Sekundärspiegel 3 ist eine erhabene Spiegelfläche und - wie in Fig. 1 angedeutet - beispielweise ein Wölbspiegel, der in Fig. 2 in einer konkreten ersten Variante als sphärisch-parabolischer Spiegel 32 in zwei zueinander orthogonalen Schnitten dargestellt ist.
In Fig. 2 zeigt die linke Darstellung einen Schnitt in der (rechts definierten) Ebene B-B durch die Mittelachse des Hauptreflektors 1 in seiner (horizontalen) Längsrichtung. In dieser Schnittebene B - B weist der Spiegel 32 eine sphärische Schnittform auf.
Die rechte Darstellung in einer zur Ebene B - B orthogonalen Schnittebene A - A des Hauptreflektors 1 zeigt den Spiegel 32 mit einer parabolischen Schnittform. Durch diese Gestaltung des Sekundärspiegels 3 wird das aus dem Lichtleiter 41 austretende Lichtbündel auf den Hauptreflektor 1 optimal abgelenkt und gleichmäßig verteilt, wenn der Scheitelpunkt des sphärisch-parabolischen Spiegels 32 im Brennpunkt des Hauptreflektors 1 liegt. Die Halterung 31 ist dabei im Randbereich der parabolischen Schnittform des Spiegels 32 nahezu parallel zur optischen Achse 11 wegen der kurzen Querausdehnung des Hauptreflektors 1 ohne wesentliche Beeinträchtigung der übertragenen Lichtstärke realisierbar.
Die Halterungs- und Bewegungselemente 2 bestehen üblicherweise aus Handgriffen 21, die in der Längsrichtung am Rand neben dem Hauptreflektor 1 zur Ausführung von Schwenkbewegungen angebracht sind, einem Reflektorträger 22, an dem die Handgriffe 21 und der Hauptreflektor 1 befestigt sind und einer Gabel 23 zur Erlangung der vertikalen Schwenkbarkeit des im Reflektorträger 22 gehaltenen Hauptspiegels 1. Im Reflektorträger 22 wird der Lichtleiter 41 in einer Passbohrung 24 gehaltert und mit wenigstens einem Gewindestift 25 gegen Verdrehen und axiale Verschiebung fixiert.
Des Weiteren ist am Reflektorträger 22 durch eine Manschette 12 hindurch, die das üblicherweise zur Durchführung der Halterungen der lichterzeugenden Komponenten vorgesehene Mittenloch des Hauptspiegels 1 verschließt, die Halterung 31 für den Sekundärspiegel 3 befestigt. Mit der Halterung 31 wird zugleich der Hauptreflektor 1 am Reflektorträger 22 mittig fixiert. Das aufgespreizte Ende der Gabel 23 der Halterungs- und Bewegungselemente 2 bietet zwischen den zwei zur schwenkbaren Verbindung der Gabel 23 mit dem Reflektorträger 22 vorgesehenen Achsbolzen 26 genügend Raum für die Durchbiegung des relativ dicken Lichtleiters 41 (mit einem Durchmesser, der ca. 10 bis 18 mm betragen kann) während einer Schwenkbewegung des Hauptreflektors 1 mit dem Reflektorträger 22.
Die Gabel 23 ist in ihrem Hauptteil hohl ausgeformt, um den aus Richtung des entfernt (z.B. im Sockel eines zahnärztlichen Behandlungsstuhles oder aber am üblichen Schwenkarm der Dentalleuchte) angeordneten Lichtgenerators 4 zugeführten Lichtleiter 41 aufzunehmen und dem Reflektorträger 22 zuzuführen. Unterliegt der Lichtleiter 41 außerhalb der Gabel 23 (z.B. über den üblicherweise vorhandenen Schwenkarm einer Dentalleuchte) einer hohen Bewegungsbeanspruchung, weist die Gabel 23 in ihrem zur Kabelführung hohl ausgeformten Hauptteil eine starke Umlenkung 27 für den Lichtleiter 41 eine Zugentlastung auf. Der durch die Zugentlastung 27 der Gabel 23 eingeführte Lichtleiter 41 ist in der Fig. 2 (und in den nachfolgenden Figuren 3 bis 5) abgeschnitten dargestellt, stellt jedoch real eine durchgängige optische Verbindung her mit dem ebenfalls abgebrochen gezeichneten Lichtleiter 41 von einem oder mehreren in den Figuren 6 und 7 dargestellten entfernt angeordneten Lichtgeneratoren 4. Ist der Lichtgenerator 4 nicht so weit entfernt und somit relativ nahe am Schwenkarm einer herkömmlichen Dentalleuchte angebracht, kann - wie z.B. in Fig. 1 angedeutet und in Fig. 8 und 9 konkret gezeigt - die Zugentlastung 27 entfallen.

In Fig. 3 ist eine zweite Variante für die Ausführung des Sekundärspiegels 3 angegeben. Die Schnittebenen A - A und B - B sind dieselben wie in Fig. 2.
Der Sekundärspiegel 3 hat in diesem Beispiel die Form einer halben Zylindermantelfläche. Der so geformte zylindrische Sekundärspiegel 33 ist mit seiner Achse parallel zur kürzeren Querausdehnung des Hauptreflektors 1 ausgerichtet. Seine die optische Achse 11 des Hauptreflektors 1 schneidende Mantelfläche, die der Austrittsfläche des Lichtleiters 41 am nächsten liegt, ist zwischen Scheitelpunkt und Brennpunkt des Hauptreflektors 1 nahe dessen Brennpunkt angeordnet. Infolge des leicht kegelförmig aus dem Lichtleiter 41 austretenden Lichtbündels wird der Hauptreflektor 1 auch in seiner Querausdehnung noch ausreichend gleichmäßig beleuchtet. Die gewölbte Schnittform des zylindrischen Sekundärspiegels 33 sorgt für die hinreichend weitgreifende Verteilung des aus dem Lichtleiter 41 austretenden Lichts in Längsausdehnung des Hauptreflektors 1.
Alle übrigen Komponenten der gesamten Beleuchtungsanordnung sind in gleicher Weise, wie zu Fig. 2 beschrieben, angeordnet.

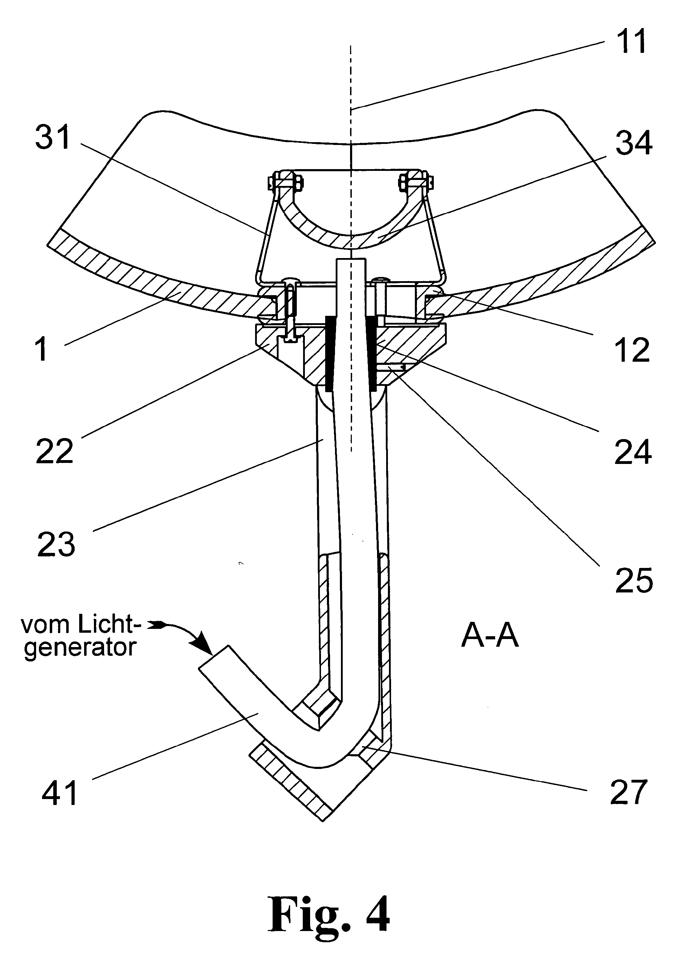

Fig. 4 enthält eine dritte Variante des Sekundärspiegels 3. Die analog zur rechten Darstellung von Fig. 3 in der Schnittebene A - A verlaufende horizontale Schnittzeichnung stellt den Hauptreflektor 1 in seiner elliptischen Schnittform dar.
Als Sekundärspiegel 3 ist in diesem Beispiel ein konvexer sphärischer Spiegel 34, der mit seinem Scheitelpunkt zwischen Scheitelpunkt und Brennpunkt des Hauptreflektors 1, sehr nahe am Brennpunkt des Hauptreflektors 1 liegt. Auch mit dieser Konfiguration aus Lichtleiter 41, sphärischem Sekundärspiegel 34 und Hauptreflektor 1 wird das aus dem Lichtleiter 41 austretende Licht hinreichend gleichmäßig auf den Hauptreflektor 1 verteilt und in die Beleuchtungsebene abgebildet. Die Anbringung und Funktion der übrigen Komponenten ist analog der Darstellung von Fig. 2 und der zugehörigen Beschreibung beibehalten.

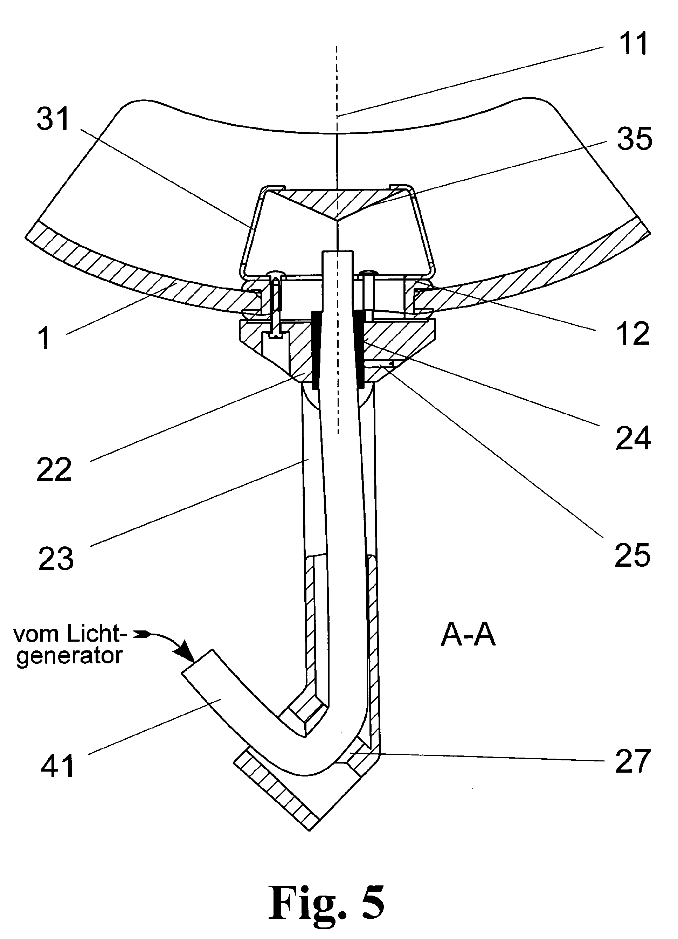

Eine besonders einfache Ausführung des Sekundärspiegels 3 ist in einem Beispiel gemäß Fig. 5 realisiert. In diesem Fall ist der Sekundärspiegel 3 die Oberseite einer Kegelmantelfläche. Ein solcher kegelförmiger Sekundärspiegel 35 zeichnet sich durch eine geringe Höhe des Kegels aus. Der Neigungswinkel der Mantelfläche des Kegels gegenüber der Grundfläche kann zwischen 10° und 35° betragen, er beträgt vorzugsweise 15°. In dieser Ausführungsform wird der Sekundärspiegel 35 mit der Kegelspitze zwischen Scheitel- und Brennpunkt des Hauptreflektors 1 etwas weniger nahe dem Brennpunkt des Hauptreflektors 1 angeordnet. Damit ist eine einfache Herstellungsform für den Sekundärspiegel 3 geschaffen, die ausreichend gut die Lichtverteilung des aus dem Lichtleiter 41 austretenden Lichtbündels auf dem Hauptreflektor 1 und somit die geforderte Ausleuchtung der gewünschten Beleuchtungsebene in ca. 700 mm Entfernung vom Hauptreflektor 1 bewirkt. Der kegelförmige Sekundärspiegel 35 ist im Gegensatz zu den vorherigen Varianten in diesem Beispiel nicht seitlich angeschraubt, sondern auf einer gekröpften Halterung 32 mit seiner Grundfläche angeklebt, um den Kegel möglichst flach zu gestalten. Um ihn in gleicher Weise wie in den vorherigen Beispielen seitlich anzuschrauben, müsste er auf einen flachen Zylinder aufgesetzt oder einstückig als Zylinder mit kegelförmiger Deckfläche hergestellt werden.

Die Fig. 6 und 7 ergänzen die in den Figuren 1 bis 5 nur als Ausgang (Terminal) in Form des Endes des Lichtleiters 41 angedeuteten Lichtquelle. Letztere besteht aus einem herkömmlich verfügbaren Lichtgenerator 4, der eine ausreichend hohe Lichtleistung im sichtbaren Spektralbereich mit der gewünschten Farbtemperatur (4500 bis 6000 K) aufweisen muss. Dazu sind Bogenentladungslampen (z.B. Halogen-Metalldampflampen oder Xenonlampen), die unter hohem Druck betrieben werden, besonders geeignet, da diese neben einer hohen Lichtausbeute und einer hohen Farbtemperatur (nahe 6000 K) eine wesentlich längere Lebensdauer gegenüber herkömmlich in Dentalleuchten verwendeten Halogenlampen aufweisen.

Wegen der örtlichen Trennung der Lichterzeugung und des Lichtaustritts am Ende des Lichtleiters 41 in der Beleuchtungsanordnung sind thermische Faktoren für die Wahl der Lichtquelle nur ein sekundäres Auswahlkriterium. Die Ausblendung von IR-Anteilen des Strahlungsspektrums der Lichtquelle kann einfach (und ohne Rücksicht auf notwendige Wärmeabfuhr) durch geeignet ausgewählte Filter (insbesondere IRund/oder UV-Filter oder Bandpassfilter, aber auch ggf. auf die gewünschte Farbtemperatur abgestimmte Farbausgleichsfilter) realisiert werden.
Der Lichtgenerator 4 hat - wie in Fig. 6 dargestellt - ein geschlossenes Gehäuse, in dem sich auch entsprechende für anspruchsvolle Lichtquellen typische Zusatzeinrichtungen (Kühleinrichtungen, Kollektoroptiken, Filter etc.) befinden. Durch die Verwendung einer derartigen Kaltlichtquelle wird (fast) keine Wärmestrahlung in den Lichtleiter 41 eingekoppelt, weil sich an der Auskoppelstelle des Lichtleiters standardmäßig ein Infrarotfilter befindet, welcher unerwünschte Wärmestrahlung ausfiltert. Sämtliche Wärmestrahlung verbleibt also im Lichtgenerator 4, wo sie mit entsprechenden Vorrichtungen (z.B. Ventilator oder andere Kühleinrichtungen) abgeleitet wird. Da sich der in Fig. 6 dargestellte Lichtgenerator 4 vorzugsweise im Fuß des Behandlungsstuhles befindet, wird die Wärmeentwicklung hier nicht als störend empfunden. An der Stelle der Lichtauskopplung aus der eigentlichen Beleuchtungseinheit in den Lichtleiter 41 steht also Licht ohne störende Infrarotanteile zur Verfügung.
Zur Steuerung der Lichtquelle weist der Lichtgenerator 4 eine Fernbedienung 42 auf, die handbedienbar in der Nähe der Handgriffe des Hauptreflektors 1 angeordnet ist. Die Energieversorgung für den Lichtgenerator 4 erfolgt vom oder im Sockel des Patientenstuhles. Die Lichtauskopplung und -weiterleitung zur Beleuchtungsanordnung (Dentalleuchte) geschieht mittels des Lichtleiters 41, der zur Übertragung einer hinreichend großen Lichtstärke zum Hauptreflektor 1 einen Durchmesser von wenigstens 10 mm aufweist. Realisierbar sind Durchmesser von Lichtleitern 41 bis maximal 24 mm, ohne wesentliche Änderungen der Bauform herkömmlicher Dentalleuchten vornehmen zu müssen. Als Lichtleiter 41 finden kommerziell verfügbare Lichtleiter (z.B. LBM Kunststoff-Endlicht EURO-FSPT, LINOS LMA Glasfaser, Flüssiglichtleiter LINOS LLG VIS, o.ä.) Anwendung. Kommerziell verfügbare Faserbündel sind dabei den Flüssigkeitslichtleitern vorzuziehen, da erstere im Bereich der Endbefestigung am Reflektorträger 22 beweglicher sind, um den Hauptreflektor 1 leichter ausrichten zu können.

Fig. 7 zeigt eine Variante für die Lichterzeugung, bei der zwei Lichtgeneratoren 4 zum Einsatz kommen. Diese Ausgestaltung dient dem Zweck, die Lichtleistung zu erhöhen, um preiswertere Leuchtmittel (wie z.B. Halogenlampen) einsetzen zu können. Derselbe Effekt kann auch mit zwei Lichtquellen in nur einem Lichtgenerator 4 erzielt werden.
Die Stromversorgung erfolgt wie in Fig. 7 über einen Netzanschluss für jeden Lichtgenerator 4. Die beiden Lichtgeneratoren 4 werden dabei simultan betrieben und über eine gemeinsame Fernbedienung 42 gesteuert. Die in den beiden Lichtgeneratoren 4 erzeugte Lichtleistung wird in Faserbündel 43 eingespeist, die aus Einzelfasern zusammengesetzt (z.B. LBM EURO-FSPT 300, LINOS GAX 12/2) sind. Die Faserbündel 43 werden dann zu einem Lichtleiter 41 zusammengeflochten, um das Licht in der oben beschriebenen Weise dem Hauptreflektor 1 zuzuführen.

In einer kostengünstigen Variante, die in Fig. 8 dargestellt ist, hat der Lichtgenerator 4 zwar herkömmliche Komponenten, ist jedoch speziell angepasst, um in kurzer Entfernung hinter dem Hauptreflektor 1 am üblich vorhandenen Schwenkarm einer Dentalleuchte befestigt zu werden. Diese Anordnung bietet vor allem den Vorteil, dass die notwendige Länge des Lichtleiters 41 und somit die im Lichtleiter 41 entstehenden Dämpfungsverluste gering gehalten werden können. Dadurch kommt für diese Gestaltungsform wiederum der Einsatz von Halogenstrahlern als Leuchtmittel in Betracht. Des Weiteren können - ebenfalls unter Kostengesichtspunkten - auch größere Durchmesser des Lichtleiters 41 verwendet werden.
In Fig. 8 ist eine von den vorherigen Gestaltungen (Einsatz kompakter Lichtgeneratoren 4 im Sockel des Patientenstuhles) abweichende Ausführung des Lichtgenerators 4 gezeigt.
Dabei sind Hauptreflektor 1, Sekundärspiegel 3 und die Halterungs- und Bewegungselemente 2 analog zu Fig. 5 angeordnet, wobei ein kegelförmiger Sekundärspiegel 35 mittels der Halterung 31 koaxial zum Hauptreflektor 1 angebracht ist. Die Austrittsfläche des Lichtleiters 41 ist in diesem Fall koaxial zu Hauptreflektor 1 und Sekundärspiegel 35 angeordnet, im Reflektorträger 22 in einer Passbohrung 24 eingespannt und über die Gabel 23 biegebeweglich in das Gehäuse 44 des Lichtgenerators 4 eingeführt. Die Gabel 23 ist dabei direkt an das Gehäuse 44 angefügt.
Das Gehäuse 44, das vorzugsweise zylinderförmig geformt ist, enthält entlang seiner optischen Achse aufeinanderfolgend die Lichtleiterhalterung 45 für die Eintrittsfläche des Lichtleiters 41, ein IR-Filter 46, das mit weiteren Filtern zur Einstellung der gewünschten Farbtemperatur ergänzt oder kombiniert sein kann, einen kommerziell verfügbaren Halogenstrahler 47 (mit Reflektor) sowie einen Lüfter 5 zur Wärmeableitung aus dem Gehäuse 44, der vorzugsweise ein Axiallüfter ist.
An einem rohrförmigen Träger 6, der eine vertikale Drehverbindung zu dem (bei herkömmlichen Dentalleuchten üblichen) Schwenkarm (nicht dargestellt) für das horizontale Ausrichten des Hauptreflektors 1 bildet, ist das Gehäuse 44 starr befestigt. Außer der in Fig. 8 gewählten horizontalen Lage der Achse der optischen Elemente des Lichtgenerators 4 ist eine Neigung des optischen Aufbaus in Richtung des Hauptreflektors 1 bis zu 30° vorteilhaft, damit beim vertikalen Schwenken des Hauptreflektors 1 um die Achsbolzen 26 der Gabel 23 herum der Lichtleiter 41 nur in möglichst großen Radien (geringe Biegebeanspruchung) gebogen wird. Auch bei dieser Ausführung ist das Leuchtmittel ein Halogenstrahler 47, der beim Bersten des Lampenkolbens im Gehäuse 44 des Lichtgenerators 4 sicher gekapselt ist. Des Weiteren wird die entstehende Wärmestrahlung vor Eintritt in den Lichtleiter 41 durch das IR-Filter 46 wirksam abgeschirmt und erwärmte Luft über den Axiallüfter 5 zur Abstrahlungsrichtung des Lichts entgegengesetzt abgeführt.

Eine weitere lichtstarke und kostengünstige Gestaltung der Erfindung zeigt Fig. 9. Hier ist als Lichtleiter 41 ein starrer Lichtleitstab 49 eingesetzt, der eine bis zu 45 % höhere Lichttransmission gegenüber Faserbündeln gleichen Durchmessers erreicht. Solche Lichtleitstäbe bestehen aus sehr vielen dünnen starren Glasfasern (Durchmesser < 1 mm), welche im Fertigungsprozess verklebt und verschmolzen werden. Sie sind bereits als Querschnittswandler in der Endoskopie oder als Übertragungsoptik bei Photopolymerisationslampen bekannt und werden z.B. als "Lichtleitstäbe für den Hochtemperaturbereich" von der Firma Faseroptik Jena GmbH oder als "Fiber rods" (gebogene Endoptik für dentale Photopolimerisationslampen) von Schott Fiberoptics Ltd. angeboten.
In diesem Beispiel ist der Lichtgenerator 4 unmittelbar hinter dem Hauptreflektor 1 positioniert und im Unterschied zu Fig. 8 direkt mit dem Reflektorträger 22 des Hauptreflektors 1 verbunden. Das Gehäuse 44 des Lichtgenerators 4 ist dabei auf den speziell angepassten Reflektorträger 22 aufgesteckt. Der Reflektorträger 22 weist - wie in allen vorherigen Beispielen - eine Passbohrung 24 auf, in die in diesem Fall der Lichtleitstab 49 eingesteckt ist. Der Lichtleitstab 49 besitzt eine sehr hohe numerische Apertur (NA = 80°) und endet deshalb sehr nahe vor dem kegelförmigen Sekundärspiegel 35. In der entgegengesetzten Richtung befindet sich vor dem Lichtleitstab 49 das IR-Filter 46, dem sich auf der optische Achse 11 vorgeordnet ein Halogenstrahler 47 und ein Lüfter 5 anschließen.
Der Halogenstrahler 47 ist über seinen zugehörigen Reflektor mittels einer Strahlerhalterung 48 am Reflektorträger 22 des Hauptreflektors 1 befestigt, wobei die Strahlerhalterung 48 ein Ausklinken des Halogenstrahlers 47 für den Leuchtmittelaustausch erlaubt.
Die vertikal schwenkbare Befestigung der gesamten Dentalleuchte mit Hauptreflektor 1, Sekundärspiegel 35 und Lichtgenerator 4 erfolgt mittels der üblichen Ankopplung über die Achsbolzen 26, wobei die Gabel 23 (nicht sichtbar) in ein Verbindungsrohr 62 übergeht, das über einen rohrförmigen Träger 6 mittels des Führungsbolzens 61 mit dem üblich vorhandenen Schwenkarm der Dentalleuchte in horizontal drehbarer Verbindung steht. Durch das Verbindungsrohr 62 hindurch wird die Stromversorgung und der Fernbedienungsanschluss in das Gehäuse 44 geleitet, wobei die Fernbedienung 42 vorteilhaft im rohrförmigen Träger 6 angeordnet ist. Die Fernbedienung 42 besteht in diesem Fall aus einem koaxial im Träger 6 integrierten Drehpotentiometer, das zum Dimmen des Halogenstrahlers 47 mittels eines kleinen Hebels von außen einstellbar ist, wobei der Hebel zusätzlich einen am äußeren Ende befindlichen Ein-/Aus-Schalter aufweist.
Alle übrigen Komponenten der Dentalleuchte, wie beispielweise die Halterungs- und Bewegungselemente 2 (hier aus Gründen der Übersichtlichkeit weggelassen), sind wie zu den vorherigen Figuren beschrieben, in gleicher Weise vorhanden und funktionieren entsprechend. Die einzige Ausnahme bildet die Zugentlastung 27 (nur in Fig. 2, 3, 4 und 5) für den Lichtleiter 41, die ersatzlos entfällt, da der Lichtleitstab 49 starr gegenüber dem Hauptreflektor 1 befestigt ist und somit die (vertikalen) Schwenkbewegungen mitmacht.

Durch den Einsatz wenigstens eines vom Innenraum des Hauptreflektors 1 separierten Lichtgenerators 4, die Zuleitung der Lichtenergie über einen Lichtleiter 4 und den geeignet gestalteten Sekundärspiegel 3 zur Verteilung des Lichts auf den Hauptreflektor 1 ergibt sich als wesentlicher Vorteil, dass sich die Dentalleuchte selbst nicht mehr erhitzt, so dass keine lästige Wärme im Nacken des Operateurs (Zahnarztes) entsteht. Des Weiteren besteht keine Notwendigkeit mehr, die Gefahr explodierender Leuchtmittel durch Splitterschutzmaßnahmen zu mindern oder durch letztere Schutzmaßnahmen eine Abdunklung oder Verzerrung des Lichtfeldes in Kauf nehmen zu müssen.

### Bezugszeichenliste

- 1: Hauptreflektor
- 11: optische Achse
- 12: Manschette

- 2: Halterungs- und Bewegungselemente
- 21: Handgriff
- 22: Reflektorträger
- 23: Gabel
- 24: Passbohrung
- 25: Gewindestift
- 26: Achsbolzen
- 27: Zugentlastung

- 3: Sekundärspiegel
- 31: Halterung
- 32: sphärisch-parabolischer Spiegel
- 33: zylinderförmiger Sekundärspiegel
- 34: sphärischer Sekundärspiegel
- 35: kegelförmiger Sekundärspiegel

- 4: Lichtgenerator
- 41: Lichtleiter
- 42: Fernbedienung
- 43: Faserbündel
- 44: Gehäuse
- 45: Lichtleiterhalterung
- 46: IR-Filter
- 47: Halogenstrahler
- 48: Strahlerhalterung
- 49: Lichtleitstab

- 5: Lüfter

- 6: rohrförmiger Träger
- 61: Führungsbolzen
- 62: Verbindungsrohr

## Patentansprüche

1. Anordnung zum Beleuchten eines dentalen Operationsfeldes, bei der eine Lichtquelle in einem konkav geformten Hauptreflektor zum Formen eines definierten Beleuchtungsfeldes angeordnet ist, eine Abschatteinrichtung zur Vermeidung direkter Lichtabstrahlung von der Lichtquelle ins Beleuchtungsfeld sowie Halterungs- und Bewegungselemente, die am Hauptreflektor zur Ausrichtung des Beleuchtungsfeldes angebracht sind, vorhanden sind, **dadurch gekennzeichnet, dass**
- die Lichtquelle eine Austrittsfläche eines Lichtleiters (41) ist, der von hinten in den Innenraum des Hauptreflektors (1) geführt ist, wobei der Lichtleiter (41) mit einem außerhalb des Hauptreflektors (1) separat angeordneten Lichtgenerator (4) in Verbindung steht und die Austrittsfläche des Lichtleiters (41) mit ihrer Flächennormalen auf die Abschatteinrichtung ausgerichtet ist, und
- die Abschatteinrichtung als Sekundärspiegel (3) ausgeführt ist, wobei der Sekundärspiegel (3) erhaben geformt und auf der optischen Achse (11) des Hauptreflektors (1) angeordnet ist, so dass ein aus dem Lichtleiter (41) austretendes Lichtbündel vollständig vom Sekundärspiegel (3) reflektiert und vom Hauptreflektor (1) zu dem definierten Beleuchtungsfeld geformt wird.

2. Anordnung nach Anspruch 1, **dadurch gekennzeichnet, dass**
der Sekundärspiegel (3) ein konvexer sphärischer Spiegel (34) ist, wobei der sphärische Spiegel (34) zwischen dem Scheitelpunkt und dem Brennpunkt nahe dem Brennpunkt des Hauptreflektors (1) angeordnet ist, so dass das aus dem Lichtleiter (41) austretende Licht den Hauptreflektor (1) im Wesentlichen vollständig ausleuchtet.

3. Anordnung nach Anspruch 1, **dadurch gekennzeichnet, dass**
der Sekundärspiegel (3) ein sphärisch-parabolischer Spiegel (32) ist, wobei der sphärisch-parabolische Spiegel (32) in einer Schnittebene, die der Längsausdehnung des Hauptreflektors (1) entspricht, parabolisch und in der dazu orthogonalen Schnittebene, die der kürzeren Querausdehnung des Hauptreflektors (1) entspricht, sphärisch geformt ist, und zwischen dem Scheitelpunkt und dem Brennpunkt des Hauptreflektors (1) so angeordnet ist, dass das aus dem Lichtleiter (41) austretende Licht den Hauptreflektor (1) im Wesentlichen vollständig ausleuchtet.

4. Anordnung nach Anspruch 1, **dadurch gekennzeichnet, dass**
der Sekundärspiegel (3) ein konvexer zylindrischer Spiegel (33) ist, wobei der zylindrische Spiegel (33) einen gegenüber seiner Höhe im Wesentlichen gleich großen Durchmesser aufweist und mit seiner Achse parallel zur kurzen Querrichtung des Hauptreflektors (1) so angeordnet ist, dass eine als Scheitel wirkende Mantellinie des zylindrischen Spiegels zwischen Scheitelpunkt und Brennpunkt des Hauptreflektors (1) nahe dessen Brennpunkt angeordnet ist.

5. Anordnung nach Anspruch 1, **dadurch gekennzeichnet, dass**
der Sekundärspiegel (3) ein kegelförmiger Spiegel (35) ist, wobei der kegelförmige Spiegel (35) mit seiner Spitze zwischen dem Scheitelpunkt und dem Brennpunkt des Hauptreflektors (1) angeordnet ist und das aus dem Lichtleiter (41) austretende Lichtbündel aufgrund seiner kegelförmig divergierenden Bündelform auf den Hauptreflektor (1) verteilt wird.

6. Anordnung nach Anspruch 1, **dadurch gekennzeichnet, dass**
der Lichtleiter (41) koaxial zur optischen Achse (11) des Hauptreflektors (1) und durch den Hauptreflektor (1) hindurch angeordnet ist, wobei der Sekundärspiegel (3) ebenfalls koaxial zum Hauptreflektor (1) positioniert ist.

7. Anordnung nach Anspruch 1, **dadurch gekennzeichnet, dass**
der Lichtleiter (41) außerhalb der optischen Achse (11) des Hauptreflektors (1) auf den Sekundärspiegel (3) gerichtet ist.

8. Anordnung nach Anspruch 7, **dadurch gekennzeichnet, dass**
der Lichtleiter (41) außerhalb der optischen Achse (11) des Hauptreflektors (1) entlang einer Schnittebene, die den Hauptreflektor (1) symmetrisch teilt, auf den Sekundärspiegel (3) gerichtet ist.

9. Anordnung nach Anspruch 7, **dadurch gekennzeichnet, dass**
der Lichtleiter (41) außerhalb der optischen Achse (11) und außerhalb der Spiegelfläche des Hauptreflektors (1) auf den Sekundärspiegel (3) gerichtet ist.

10. Anordnung nach Anspruch 1, **dadurch gekennzeichnet, dass**
der Lichtleiter (41) aus einzelnen Fasern zusammengesetzt ist.

11. Anordnung nach Anspruch 10, **dadurch gekennzeichnet, dass**
der Lichtleiter (41) aus einzelnen Fasern zusammengesetzt ist, wobei die Faserbündel (43) von wenigstens zwei Lichtgeneratoren (4) zu einem Lichtleiter (41) zur Einkopplung am Hauptreflektor (1) zusammengefügt sind.

12. Anordnung nach Anspruch 10, **dadurch gekennzeichnet, dass**
der Lichtleiter (41) aus einzelnen Fasern zusammengesetzt ist, wobei die Faserbündel (43) von wenigstens zwei Lichtquellen innerhalb eines Lichtgenerators (4) zu einem Lichtleiter (41) zur Einkopplung am Hauptreflektor (1) zusammengefügt sind.

13. Anordnung nach Anspruch 11 oder 12, **dadurch gekennzeichnet, dass**
bei Zusammenführung von Fasern (44) aus mehreren simultan betriebenen Lichtgeneratoren (4) Halogenlampen als Leuchtmittel einsetzbar sind.

14. Anordnung nach Anspruch 1, **dadurch gekennzeichnet, dass**
bei Verwendung eines langen Lichtleiters (41) von einem Lichtgenerator (4) mit nur einem Leuchtmittel zum Hauptreflektor (1) eine Bogenentladungslampe als Leuchtmittel eingesetzt ist.

15. Anordnung nach Anspruch 1, **dadurch gekennzeichnet, dass**
bei Verwendung eines sehr kurzen Lichtleiters (41) von einem Lichtgenerator (4), der mit nur einem Leuchtmittel ausgestattet und nahe dem Hauptreflektor (1) angeordnet ist, der Lichtgenerator (4) einen herkömmlichen Halogenstrahler (47) mit Reflektor und nachgeordnetes IR-Filter (46) enthält.

16. Anordnung nach Anspruch 15, **dadurch gekennzeichnet, dass**
der Lichtgenerator (4) am herkömmlichen Schwenkarm zur horizontalen Einstellung des Hauptreflektors (1) horizontal angebracht ist.

17. Anordnung nach Anspruch 15, **dadurch gekennzeichnet, dass**
der Lichtgenerator (4) am herkömmlichen Schwenkarm zur horizontalen Einstellung des Hauptreflektors (1) bis zu 30° in Richtung des Hauptreflektors (1) abwärts geneigt angebracht ist, um die Krümmung des Lichtleiters (41) zu minimieren.

18. Anordnung nach Anspruch 15, **dadurch gekennzeichnet, dass**
der Lichtgenerator (4) unmittelbar hinter dem Hauptreflektor (1) entlang der verlängerten optischen Achse (11) des Hauptreflektors (1) angebracht ist, wobei als Lichtleiter (41) ein starrer Lichtleitstab (49) eingesetzt ist und das Gehäuse (44) des Lichtgenerators (4) mit dem Reflektorträger (22) des Hauptreflektors (1) schwenkbar ist.
